(19) Europäisches Patentamt
European Patent Office
Office européen des brevets

(11) Publication number: **0 137 646**
**A1**

(12) # EUROPEAN PATENT APPLICATION

(21) Application number: 84305532.8

(22) Date of filing: 14.08.84

(51) Int. Cl.⁴: C 12 N 15/00
C 12 P 13/04, C 12 P 13/22
//C12R1/19

(30) Priority: 16.08.83 US 523551

(43) Date of publication of application:
17.04.85 Bulletin 85/16

(84) Designated Contracting States:
AT BE CH DE FR GB IT LI LU NL SE

(71) Applicant: GENENTECH, INC.
460 Point San Bruno Boulevard
South San Francisco California 94080(US)

(72) Inventor: Lawlis, Virgil Bryan
1285 Alicante Drive
Pacifica California 94044(US)

(72) Inventor: Rastetter, William Harry
818 N. Odaho Street
San Mateo California 94401(US)

(72) Inventor: Snedecor, Bradley Richard
223 Tulare Street
Brisbane California 94005(US)

(74) Representative: Goldin, Douglas Michael et al,
J.A. KEMP & CO. 14, South Square Gray's Inn
London WC1R 5EU(GB)

(54) **Recombinant process for preparing L-amino acids, recombinant expression vectors and transformed microorganisms for use in the process.**

(57) A process for preparing an L-amino acid which comprises reacting the corresponding α-keto acid with ammonia, in the presence of a compatible source of reducing equivalents, and in the presence of a recombinant microorganism transformed with at least one recombinant expression vector encoding one or more enzymes useful in the process and which enzymes are selected from the group consisting of dehydrogenase and transaminase enzymes of suitable specificity.

EP 0 137 646 A1

0137646

# RECOMBINANT PROCESS FOR PREPARING L-AMINO ACIDS, RECOMBINANT EXPRESSION VECTORS AND TRANSFORMED MICROORGANISMS FOR USE IN THE PROCESS

## Background of the Invention

The field of this invention is the production of amino acids in recoverable quantities. In particular, the invention relates to a process for producing L-amino acids employing the metabolism of a recombinant microorganism using as starting materials the corresponding α-keto acid and ammonia.

Amino acids are useful in a variety of applications, including direct nutritional supplements, food additives, and in intraveneous feeding. A more recent application of specific amino acids is the sweetener Aspartame which is composed of phenylalanine methyl ester and aspartic acid.

The two most common approaches used in preparing amino acids are chemical synthesis and bacterial fermentation. With respect to the former, the necessity for controlling the stereochemistry of the product poses a serious problem which cannot easily be overcome by present techniques. With respect to the fermentation approach, commonly the carbon source for the backbone of the amino acid is the

foodstuff for the microorganism or some other distantly related metabolite. Accordingly, specialized organisms containing the appropriate complex metabolite are required, and the yields are often less than desired. Recently, a stereoselective chemical process which comprises fermenting a hydantoin compound structurally related to the desired L-amino acid has been disclosed (see U.S. Patent 4,016,037).

The present invention capitalizes on stereoselective enzyme catalyzed reactions introduced or amplified by transforming a host microorganism, and focuses on the direct conversion of an α-keto acid to the corresponding L-amino acid using ammonia. In this reaction, of course, it is necessary to supply reducing equivalents in order to adjust the oxidation state of the substrate α-keto acid. Advantage is taken of the ability of the host organism to provide these reducing equivalents through its normal metabolic pathways.

While it has been known for some time that E. coli hosts contain some of the enzymes required to carry out the processes of the present invention, the conversion is greatly enhanced by use of recombinant techniques to elevate or otherwise regulate the amounts and activities of those enzymes which specifically convert α-keto acids to the corresponding amino acids. Such enhancement occurs by optimizing the quantities of the appropriate enzymes.

## Summary of the Invention

The present invention provides a convenient, economic, and direct process for producing a desired L-amino acid. In the process of the present invention, a culture of a suitable recombinant microorganism is provided with stoichiometric amounts of the corresponding α-keto acid and ammonia. As the culture continues to utilize its usual carbon source, reducing equivalents are produced

through this metabolism to provide the necessary reducing power to carry out the transformation of the α-keto acid into the L-amino acid form catalyzed by the enzymes regulated by the introduced recombinant system. Thus, the materials consumed in the process of the invention are the α-keto acid, ammonia, and sufficient foodstuff for the organism to provide the desired reducing power. The amino acid thus prepared can be isolated from the culture medium using standard techniques.

In another aspect, the invention relates to replicable expression vectors containing DNA sequences encoding the amino acid sequences of, and capable of effecting the production of, the enzymes catalyzing the desired conversion. Still other aspects relate to a process for transforming host cells with such vectors, the resulting recombinant cells as cell cultures, and the amino acids produced by such recombinant cells.

By providing suitable amounts of the starting materials to the recombinant cells, the amino acids can be produced in commercial quantities, exceeding the level of 30g/L of cell culture, and at rates exceeding 10mg/L/OD/hr unit, where OD units are used as a measure of cell density. Furthermore yields exceed 50 percent of stoichiometry. Sufficient quantities are often produced that in the case of relatively insoluble L-amino acids, crystals of the desired product can be seen in the medium.

## Brief Description of the Drawings

Figure 1A, 1B, 1C is a graphic representation of phenylalanine production by a recombinant strain of E. coli RV308 transformed by the plasmid pA2.

Figure 2A and 2B shows the construction of an expression vector encoding glutamate dehydrogenase.

Figure 3 shows the construction of an expression vector encoding transaminase B.

Figure 4 shows the construction of the expression vector pA2 which encodes both glutamate dehydrogenase and transaminase B.

## Detailed Description

### A. Definitions

As used herein, the terms "α-keto-acid", "amino acid", and "ammonia" are intended to, and do, include these forms specifically, as well as the corresponding salts. The reactions which take place herein are conducted in the course of metabolism of the subject microorganism, and accordingly, in whatever ionization state these materials are supplied, they will be altered suitably by the medium or by the milieu provided by the organism to the correct form. Thus, the acids above may be supplied either as the free acid or as the corresponding, for example, sodium, potassium, or other convenient salt. Similarly, ammonia is clearly most conveniently supplied in its salt form, for example as ammonium sulfate, ammonium chloride, or other suitable embodiment. Finally, in addition to the aforementioned terms, specific α-keto acids or amino acids may be referred to as, either, for example, "phenylpyruvic acid" or "phenylpyruvate". No implication is intended by either of these names as to the free acid or salt form of the compound. It is recognized that these compounds in solution will be in a form dependent entirely on the pH of the surrounding solution and in crystallized form, dependent on the mode of preparation. Therefore, whichever variant of the name is used designates the organic portion of the molecule irrespective of its ionization state.

"Stoichiometric amounts" of reactants refers to amounts which are sufficient to provide quantities consumed in the reaction in question. It is meant to distinguish from "catalytic amounts" and includes instances in which one or more reactants is supplied in excess.

"Microorganism" is intended to encompass prokaryotic cells such as bacteria. Particularly, strains of E. coli are suitable as host cells in the method of the invention.

"Suitable specificity" when used in connection with an enzyme, is intended to designate a capacity on the part of the enzyme to interact with particular substrates which are relevant to the context of the use of the term. For example, a transaminase for the preparation of phenylalanine which is of suitable specificity is a transaminase which will accept phenylpyruvate as a substrate and catalyze transamination with an available amino acid in the metabolism of the host organism, such as L-glutamate.

"Expression vector" refers to a vehicle containing a DNA sequence which is operably linked to a suitable control sequence capable of effecting the expression of said DNA in a suitable host. Such control sequences include a promoter to effect transcription, optionally an operator sequence to control such transcription, codons for a suitable ribosome binding site, and sequences which control termination of transcription and translation. The vector may be a plasmid, a phage particle, or simply a potential genomic insert. Once transformed into a suitable host, the vector may replicate and function independently of the host genome, or may, in some instances, integrate into the genome itself. In any case, its functionality would be retained. In the present specification, "plasmid" and "vector" are sometimes used interchangeably as the plasmid is the most commonly used form of vector at present. However, the invention is intended to include such other forms of expression vectors which serve equivalent functions and which are, or become, known in the art.

DNA sequences which are "operably linked" refers to DNA sequences which are juxtaposed in such a way that their respective functions are mutually dependent. For example, a promoter operably linked to a coding sequence is capable of effecting the expression

of the coding sequence. An operator which is operably linked to a promoter is capable, when bound to a repressor protein, of inhibiting the function of the promoter, and when derepressed of permitting it to function normally. Such an operator sequence may overlap the promoter sequence or may lie downstream from it; "operably linked" is independent of such location as long as the functional interrelationship between the two sequences is maintained.

"Recombinant cells" refers to cells which have been transformed or transfected with vectors constructed using recombinant DNA techniques; "host cells" refers to such cells before they have been subjected to such transformation. As relevant to the present invention, recombinant cells are those which produce or secrete one or more proteins each in its various forms by virtue of having been transformed with expression vectors encoding it. A polypeptide secreted in accordance with the present invention (i.e., exported into the medium supporting the host cells), can be produced in mature form, or in a form associated with residual N-terminally bound amino acids, as artifacts of expression. Such residual amino acids either do not interfere with the essential bioactivity of the protein comprising the major part of the secreted polypeptide, or are subject of removal therefrom.

B. <u>General Description</u>

The stoichiometry of the process of the present invention is summarized in the reaction:

$$R-\overset{\displaystyle O}{\overset{\|}{C}}-COOH \;+\; NH_3 \;+\; 2H^+ \;+\; 2e^- \longrightarrow R-\overset{\displaystyle NH_2}{\overset{|}{C}}HCOOH \;+\; H_2O$$

(No attempt has been made to show the proper state of ionization of any of the reagents or products, as indicated in section A above.)

The invention is not limited to use of recombinant organisms which catalyze any specific pathway. However, to illuminate the nature of the process of the invention, reference is made to enzymes known to be present in organisms used as sources for DNA sequences employed in a preferred embodiment. Consistent with the nature of these enzymes is the deduction that a suitable pathway for the overall stoichiometry is provided by combining the activities of NADP-dependent glutamate dehydrogenase and a transaminase with the capacity of the cell culture to provide reducing equivalents of NADPH. Thus, in this particular embodiment, the ammonia or ammonium salt is reacted with α-keto glutaric acid in the presence of glutamate dehydrogenase and NADPH to form glutamic acid, which is in turn reacted by transamination with the substrate α-keto acid, in this case phenylpyruvate, to form the desired L-amino acid and regenerate α-keto-glutarate. These reactions are shown below:

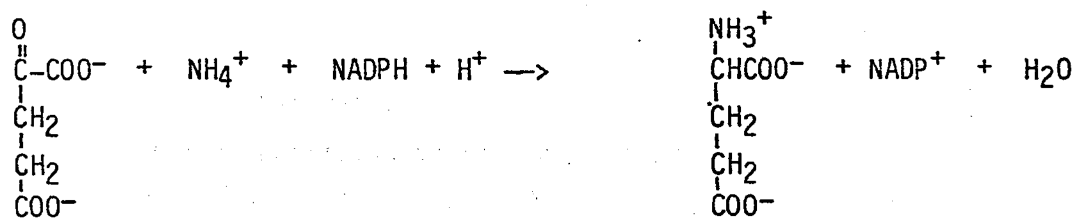

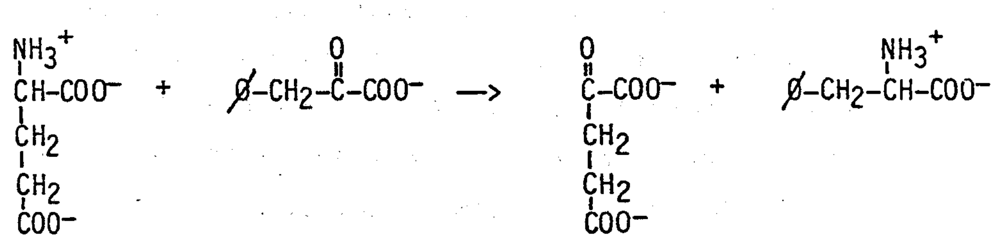

In this case, the reagents have been shown in their proper ionization state for approximately neutral pH. However, it is possible that other pH ranges could be used. Also, no implication is carried as to the form in which the materials are supplied to the organism.

It is immediately apparent that the glutamate performs merely a shuttle function in this pair of reactions, and thus stoichiometric concentrations of it are not required. NADPH (or NADH) appears to be required in stoichiometric amounts, however it is regenerated

from the $NADP^+$ (or $NAD^+$) formed by utilizing the metabolic machinery of the host cell. Typically glucose is metabolized by such cells, thus providing the reducing equivalents in the form of NADPH (or NADH). Other oxidizable substrates could also be used so long as they are metabolized by a route which permits the electrons to be passed to $NAD^+$ (or $NADP^+$). Thus, in terms of cofactor requirements, regeneration is effected at the expense only of the host cells' electron source. Furthermore, in addition to electron sources ordinarily used by the cell, alternate nutrients can utilized through providing suitable pathways also using recombinant DNA techniques. This permits the stoichiometric consumption of less expensive or more convenient substrates. For example, additional transformation of the host with an expression vector encoding formate dehydrogenase would permit formate to be used as the source of reducing equivalents.

The process of the reaction is carried out by growing a suitable recombinant organism, preferably utilizing as host an E. coli, and most preferably E. coli K12 strain RV308 (ATCC No. 31608) on a defined medium supplemented with casein amino (casamino) acids in a shake flask, fermenter or other suitable configuration to an OD of approximately 20 to 100, preferably around 30. An α-keto acid, for example phenylpyruvate is added in a concentration range of about 5 to 10 g/L, preferably around 10 g/L; an ammonium salt, for example ammonium sulfate is then added to supplement that present in the medium in a concentration which is in stoichiometric excess with respect to the α-keto acids. These concentrations are maintained over a suitable time period such as 10 to 40 hours, preferably around 15 hours or the substrate is permitted to be depleted. The amino acid formed from the α-keto acid substrate is then recovered from the medium by standard techniques appropriate to the particular amino acid in question.

The process of the invention is capable of effecting this conversion at a rate in excess of 10 mg product/liter/hour/$OD_{600}$ and under favorable, preferred conditions in excess of 100 mg/L/OD/hr.

0370L

Yields are in excess of 50 percent total conversion of the α-keto acid to the corresponding L-amino acid, and the amounts of L-amino acid produced exceed 30 grams per liter of cell culture.

In a particularly preferred embodiment, the expression vector which is used to transform the host organism (preferably E. coli RV308) encodes, in the case of production of for example phenylalanine, both glutamate dehydrogenase and transaminase B, a transaminase whose specificity includes the capacity to shuttle ammonia between glutamic acid and phenylpyruvate. Use of the recombinant E. coli as the catalytic organism results in increased yields of the desired amino acid and enhancement of the rate of conversion.

Other workable ways to produce the desired recombinant organism include the furnishing of separate vectors, one of which encodes a transaminase, and the other a dehydrogenase, both of suitable specificity and cotransforming the same host cell or cell culture with these vectors. Alternatively, depending on the level of related enzymes normally found in the host, only one or the other of such vectors may be used. Construction of the expression vectors and transformation of host cells with them are carried out by standard methods known in the art.

The following example is intended to illustrate, but not to limit the invention. For example, by varying the nature of the transaminase gene, a wider range of α-keto acids may be used as substrates, depending on the specificity of the transaminase selected. Techniques for obtaining the genes for such transaminases are known in the art, and the appropriate transaminase gene can be substituted for transaminase B encoding DNA in the foregoing constructions. Further, other amino acid dehydrogenases may be used in place of GDH. At the present, most of such enzymes are $NAD^+$ or $NADP^+$ dependent. Thus, any such amino acid dehydrogenase will couple into the production of NADPH or NADH by the reducing equivalents generated by the host cell.

# C. Detailed Description of Preferred Embodiments

## C.1 Construction of pA2, an Expression Vector for both Transaminase and Dehydrogenase

### C.1.1 Methods Employed

Vector Construction

Construction of suitable vectors containing the desired coding and control sequences employ standard ligation techniques. Isolated plasmids or DNA fragments are cleaved, tailored, and religated in the form desired to form the plasmids required. The methods employed are not dependent on the DNA source, or intended host.

Cleavage is performed by treating with restriction enzyme (or enyzmes) in suitable buffer. In general, about 1 μg plasmid or DNA fragments is used with about 1 unit of enzyme in about 20 μl of buffer solution. (Appropriate buffers and substrate amounts for particular restriction enzymes are specified by the manufacturer.) Incubation times of about 1 hour at 37°C are workable. After incubations, protein is removed by extraction with phenol and chloroform, and the nucleic acid is recovered from the aqueous fraction by precipitation with ethanol.

If blunt ends are required, the preparation is treated for 15 minutes at 15° with 10 units of E. coli DNA Polymerase I (Klenow) to fill in, or with S1 exonuclease to cut back, then phenolchloroform extracted, and ethanol precipitated.

Size separation of the cleaved fragments is performed using 6 percent polyacrylamide gel described by Goeddel, D., et al, Nucleic Acids Res., 8: 4057 (1980) incorporated herein by reference.

For ligation, approximately equimolar amounts of the desired components, suitably end tailored to provide correct matching are treated with about 10 units T4 DNA ligase per 0.5 μg DNA. (When cleaved vectors are used as components, it may be useful to prevent religation of the cleaved vector by pretreatment with bacterial alkaline phosphatase.)

A commonly used technique for obtaining a DNA sequence cleaved at a specific location which is used frequently in the invention is the "primer repair" reaction described in US 133,296, filed March 24, 1980 incorporated herein by reference and published as EPO Application Publication No. 0036776, Sept. 30, 1981. In this reaction, the fragment of DNA desired to be specifically cleaved is denatured and mixed with a primer approximately 12 bases long, which is complementary to one of the denatured strands. The primer is constructed so that one end of the primer is exactly contiguous with the desired cleavage point. The primer can be designed so as to be complementary either to the positive or negative sense strand, thereby controlling the direction of "repair". In the repair, the mixture is treated with DNA polymerase I (Klenow fragment) which effects the synthesis of a strand complementary to the denatured, primer-bound DNA in the 5' to 3' direction starting at the 3' end of the bound primer. S1 nuclease is used to cleave back the remaining single stranded denatured portion extending from the primer's 5' end. Thus, the entire double stranded sequence from the desired blunt end is resynthesized or "repaired".

Product Identification

Phenylalanine and β-phenylpyruvate were quantitatively analyzed by reverse phase HPLC (C8) and eluted with 25 percent MeOH-75 percent 0.1 percent TFA/$H_2O$.

Phenylalanine produced in the bioconversion can be isolated and purified by means known in the art, for example by adsorption to activated carbon, elution and recrystallization from water.

0370L

Sequence Confirmation

In the examples described below correct ligations for plasmid construction are confirmed by transforming E. coli K12 strain 294 (ATCC 31446) with the ligation mixture. Successful transformants were selected by ampicillin or tetracycline resistance depending on the mode of plasmid construction. Plasmids from the transformants were then prepared, analyzed by restriction and/or sequenced by the method of Messing, et al, Nucleic Acids Res., 9:309 (1981) or by the method of Maxam, et al, Methods in Enzymology, 65:499 (1980).

Transformation

Cell transformation is carried out by calcium treatment using calcium chloride as described by Cohen, S.N. et al Proc. Natl. Acad. Sci. (USA), 69: 2110 (1972).

C.1.2 Details of Construction

a. Construction of a GDH Expression Vector

Figure 2 shows the construction of an expression vector capable of expressing the gene for glutamate dehydrogenase (GDH) under control of the trp promoter, pGDHtrpCla. The construction utilizes pSAE4 as the source of the GDH gene. pSAE4 is a subclone prepared from a plasmid described by Covarrubias, A, et al., Plasmid, 3: 150 (1980) and contains the entire prokarytic GDH gene. The trp promoter is derived from the plasmid vector phGH 207-1, described in US Appl. Serial Number 307,473, filed Oct. 1, 1981 (corresponding to EPO Publn. No. 0036776) incorporated herein by reference.

In the construction, the N-terminal end of the GDH gene is inserted into phGH 207-1 as follows: phGH 207-1 is cleaved with EcoR1 and blunt ended with polymerase I, Klenow fragment, to open the vector downstream of the trp promoter. pSAE4 is treated with TaqI, which removes the front end of the desired gene including

0370L

the start codon. The gene is tailored using a primer repair reaction wherein the primer is 5'-ATGGATCAGACATA. The resulting double stranded DNA thus begins substantially at the start codon and terminates at arbitrary point past the HpaI cleavage site in the body of the gene. Ligation of this primer repaired fragment with the vector fragment derived from phGH 207-1 gives an intermediate plasmid containing the front part of the GDH gene, pgdh(f) wherein the sequence of the junction between the trp promoter EcoR1 site and the gene reads 5'-GAATTCTATG.

pSAE4 also provides the back end of the gene and the vector backbone for the final construction. pSAE4 is digested with Pstl, followed by partial digestion with HpaI as shown in Figure 3. The large vector fragment is ligated with the smaller fragment isolated after Pstl and partial HpaI digestion of pgdh(f). The resulting plasmid, pGDHtrp, contains the entire GDH sequence under control of the trp promoter, and is capable of expressing the GDH gene when transformed into a suitable host. In a final step, one of the two Clal sites downstream from the gene is removed by Clal digestion followed by religation of the plasmid, to give pGDHtrpCla, also capable of such expression.

b. Construction of an Expression Vector for the Transaminase B Gene

The source of the transaminase B gene is a derivative of the clone of a chromosomal ilvE (isoleucine valine enzyme) region from *E. coli* described by Lawther, R.P. *et al.*, *Nucleic Acids Res.* 7: 2289 (1979), incorporated herein by reference. (ilvE is a synonym for the transaminase B gene.) Lawther describes the preparation of the plasmid pRL5 which contains a portion of the ilvE gene. pRL50 was prepared in an analogous manner to pRL5 but contains the entire ilvE gene sequence ligated into the corresponding Hind III site of pBR322. Digestion of pRL50 with DdeI followed by S1 exonuclease digestion to provide blunt ends, followed

by HindIII digestion and isolation of the ~260 base pair fragment provided the front end of the transaminase gene including the start codon. To provide the C-terminal end, pRL50 is digested with Sal I and Pvu II, which results in excision of the entire gene. The gene is ligated with Pstl linkers (GCTGCAGC, available from New England Biolabs) and then double digested with Hind III and Pst I. (See Figure 3)

Finally, the trp promoter and vector backbone are provided by the plasmid pRl described in US Patent Appl. Serial Number 452,227 (see European Patent Appln. No. 83307841.3) filed December 22, 1982, and incorporated herein by reference, by treating pRl with Xbal, blunt ending with SI exonuclease, and digesting with Pstl. The large vector fragment is then used in combination with the blunt HindIII front end portion of the transaminase gene and the HindIII Pst I C-terminal portion of the transaminase gene in a three way ligation to produce the expression plasmid pAl which contains the transaminase B (ilvE) gene under the control of the trp promoter, as shown in Figure 3. pAl, when transformed into a suitable host, is capable of effecting the production of transaminase B in the recombinant organism.

The expression plasmid pRl was constructed by incubating EcoRl-Pst I cleaved γ-IMM plasmid with the 5' and 3' segments constructed as described above in the presence of T4 ligase. (γ-IMM, is a pBR 322 derived plasmid, the construction of which is essentially that described for pIFN-γ trp 48 in EP 77670, GB 2107718, incorporated herein by reference and by Gray et al., Nature, 295: 503 (1982). Specifically, γ-IMM differs from pIFN-γ trp 48 by one base pair so as to contain an XbaI site contiguous to the EcoRI site downstream from the trp promoter. The XbaI site unaccountably lost in the construction of pIFN-γ trp 48 there described can be introduced by replacement of the ca. 40 bp sequence spanning the HpaI (within the trp promoter) to EcoRI site of pIFN-γ trp 48 with a corresponding ca. 40 bp HpaI to EcoRI sequence having an XbaI site contiguous to the EcoRI site, prepared by suitable

digestion of pLeIFA25 (Goeddel et al., Nature, 287: 411 (1980) or pHKY1 (European Patent Application Publication No. 0036776).)

c. Construction of pA2 Containing the Transaminase B and GDH Sequences

pA2 was constructed using a three way ligation of 1) the large vector fragment formed by double digestion of pA1 with Cla1 and BamH1; 2) the ~ 3300 base pair fragment obtained by double digestion of pGDHtrpCla with Pst1 and Cla, and a 3) ~ 380 base pair fragment formed from double digestion of pRIF (see next paragraph) with Pst1 and BamH1. This three way ligation is shown in Figure 4.

This fragment from pRIF is equivalent to the fragment that would be obtained by excising the EcoR1-BamH1 fragment from pBR322 (Bolivar, et al., Gene, 2:95 (1977)) and adapting the EcoR1 site of the fragment to a Pst site with the adaptor GAATTCTGCAG.

C.2 Preparation of L-Phenylalanine Using *E. coli* RV308-pA2

pA2 was used to transform *E. coli* K12 RV308 (ATCC No. 31608) according to the method of Cohen (supra). A 10 liter fermenter containing minimal medium, 25 g/L in glucose and supplemented with casamino acids was inoculated with the resultant *E. coli* RV308pA2 and grown to an OD at 550 of ~30 over a period of 12 hours. (The pH was controlled at 7.0 and the temperature at 37°C.) 1 liter of the culture was then transferred to a 1.5L fermenter, and 10 g/L (76mM) ammonium sulfate added. A 10 percent (wt/vol) of sodium phenylpyruvate (Sigma) solution was fed continuously into the fermenter, sufficient to maintain 5-10g/liter phenylpyruvate in the culture, along with 50 percent wt/vol glucose solution sufficient to maintain 28mM glucose in the culture. Ammonium sulfate was added periodically batchwise to effect excess ammonium ion concentration at approximately 5g/liter. Figure 1A shows the time course of phenylalanine production under these conditions. As there shown, the phenylalanine concentration reaches a level of ~32g/L after ~19 hours. The initial (0-4 hrs.) specific phenylalanine production rate was 120 mg/L/OD/hr. A second experiment illustrated in Figure 1B gave a higher initial rate -- 160 mg/L/OD/hr.

Figure 1C shows the results of another experiment similar except that the culture initially grown is transferred to a shake flask, rather than a fermenter. In this run, phenylalanine is produced to a level of 7.5 g/liter after two hours with an initial rate of 200 mg/L/OD/hr.

CLAIMS

1. A process for preparing an L-amino acid which process comprises:

reacting the corresponding α-keto acid with ammonia,
in the presence of a compatible source of reducing equivalents, and
in the presence of a recombinant microorganism transformed with at least one recombinant expression vector encoding one or more enzymes useful in the process and which enzymes are selected from the group consisting of dehydrogenase and transaminase enzymes of suitable specificity.

2. A process of converting an α-keto acid into the corresponding L-amino acid which process comprises: supplying a recombinant microorganism cell culture transformed with at least one recombinant expression vector encoding one or more enzymes useful in the process with the α-keto acid, with ammonia, and with a source of reducing equivalents.

3. The process of claim 1 or 2 wherein the microorganism is a strain of *E. coli*.

4. A process according to any one of the preceding claims wherein the L-amino acid is phenylalanine.

5. A process according to any one of the preceding claims wherein the source of reducing equivalents is glucose.

6. A process according to any one of the preceding claims wherein the L-amino acid is produced in quantities greater than 30 grams per liter of cell culture.

7. A process according to any one of the preceding claims wherein the L-amino acid is produced at a rate greater than 10 mg/L/OD/hr.
8. A process according to claim 7 wherein the L-amino acid is produced at a rate greater than 100mg/l/OD/hr.
9. A process according to any one of the preceding claims wherein the α-keto acid is converted to the corresponding L-amino acid in greater than 50 percent yield.
10. A process according to any one of the preceding claims wherein the enzymes encoded by a recombinant vector include a transaminase of suitable specificity.
11. A process according to any one of claims 1 to 9 wherein the enzymes encoded by a recombinant vector include an amino acid dehydrogenase of suitable specificity.
12. A process according to any one of the preceding claims wherein both a suitable transaminase and a suitable dehydrogenase are encoded by a recombinant expression vector.
13. A microorganism or culture useful in carrying out the process of any one of the preceding claims.
14. A recombinant expression vector which comprises a DNA sequence encoding an amino acid dehydrogenase operably linked to a promoter capable of effecting expression in a suitable host cell.
15. A recombinant expression vector which comprises a DNA sequence encoding a transaminase operably linked to a promoter capable of effecting expression in a suitable host cell.
16. A recombinant expression vector which comprises a DNA sequence encoding an amino acid dehydrogenase

and a transaminase each operably linked to a promoter capable of effecting expression in a suitable host cell.

17. A microorganism or culture thereof transformed with a vector according to any one of claims 14 to 16.

18. A process for preparing a microorganism capable of carrying out a process according to any one of claims 1 to 12 which comprises transforming a microorganism or a culture thereof with a vector according to any one of claims 14 to 16.

19. A process for preparing a microorganism capable of carrying out a process according to any one of claims 1 to 12 which comprises transforming a microorganism or a culture thereof with a vector according to claim 14 and a vector according to claim 15.

20. A process for preparing a microorganism capable of carrying out a process according to any one of claims 1 to 12 which comprises transforming a microorganism or a culture thereof with a vector according to any one of claims 14 to 16 or with any combination thereof.

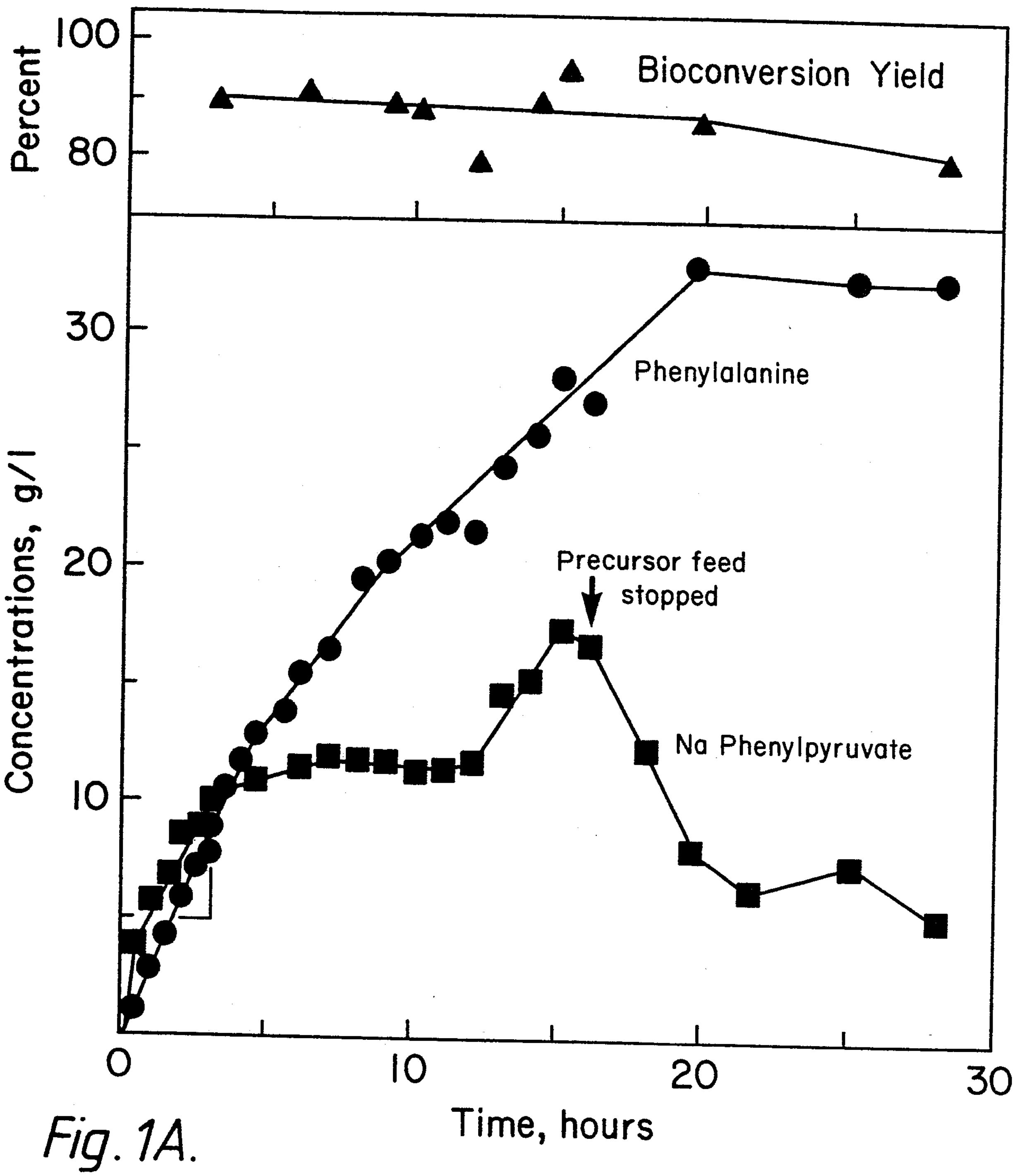
Percent
100
80
Bioconversion Yield
Concentrations, g/l
30
20
10
Phenylalanine
Precursor feed stopped
Na Phenylpyruvate
0
10
20
30
Time, hours

Fig. 1A.

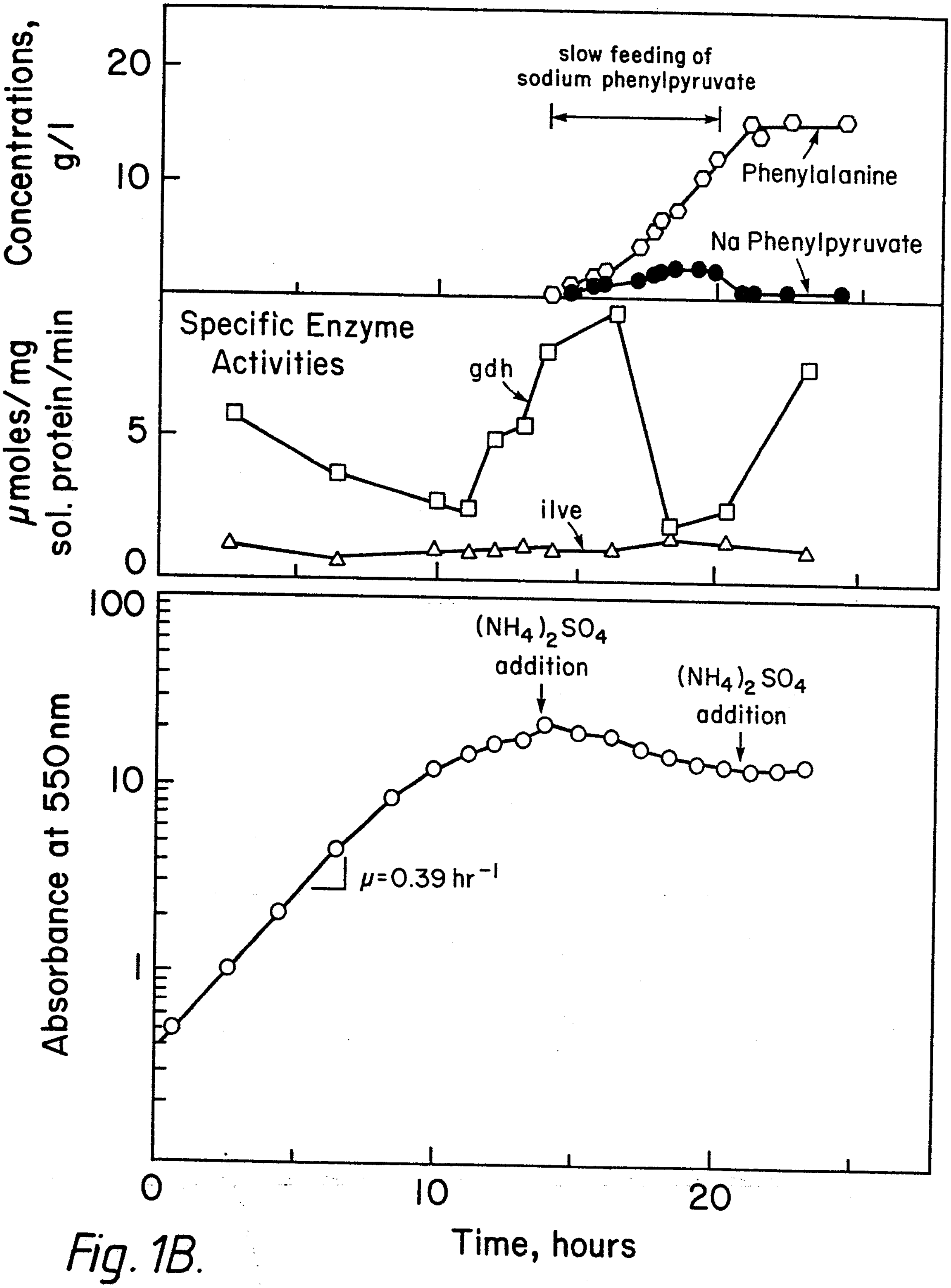
Concentrations, g/l
20
10
slow feeding of sodium phenylpyruvate
Phenylalanine
Na Phenylpyruvate
Specific Enzyme Activities
gdh
ilve
μmoles/mg sol. protein/min
5
0
100
10
1
Absorbance at 550nm
$(NH_4)_2SO_4$ addition
$(NH_4)_2SO_4$ addition
$\mu$=0.39 $hr^{-1}$
0
10
20
Time, hours

Fig. 1B.

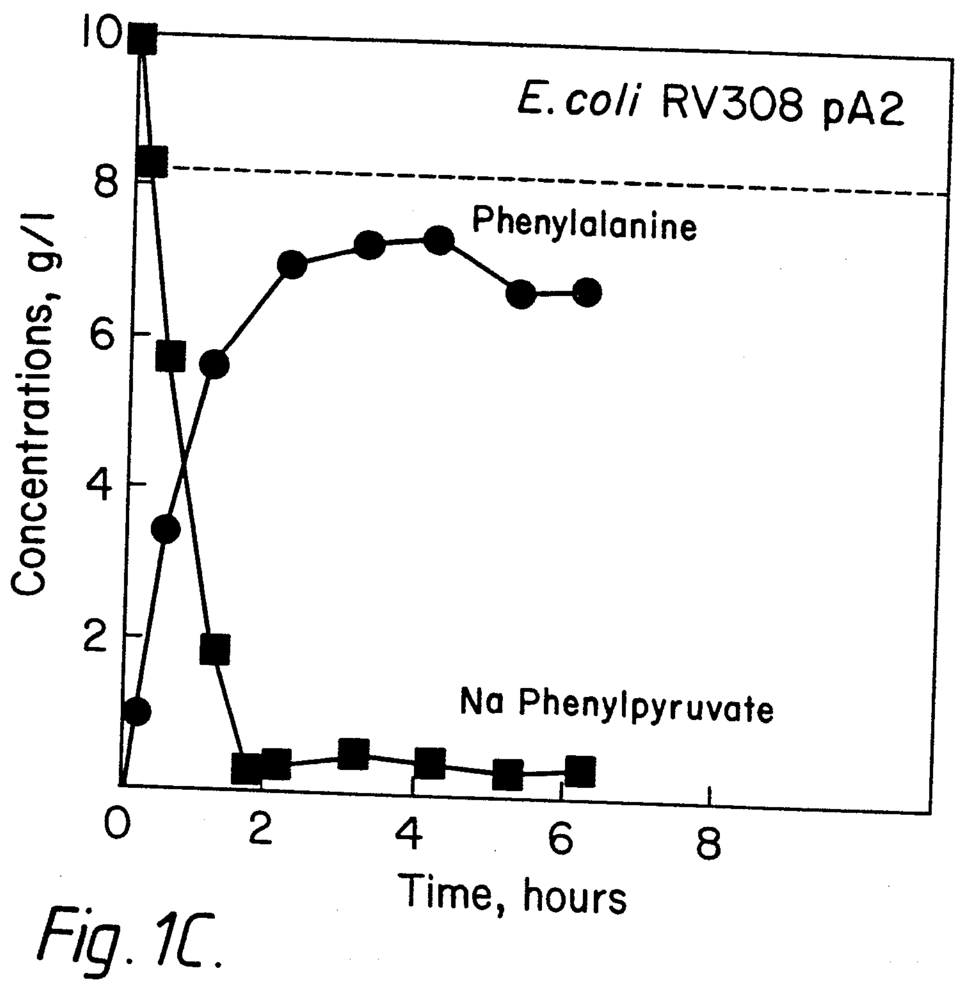
10
8
6
4
2
0
2
4
6
8
Concentrations, g/l
Time, hours
E. coli RV308 pA2
Phenylalanine
Na Phenylpyruvate

Fig. 1C.

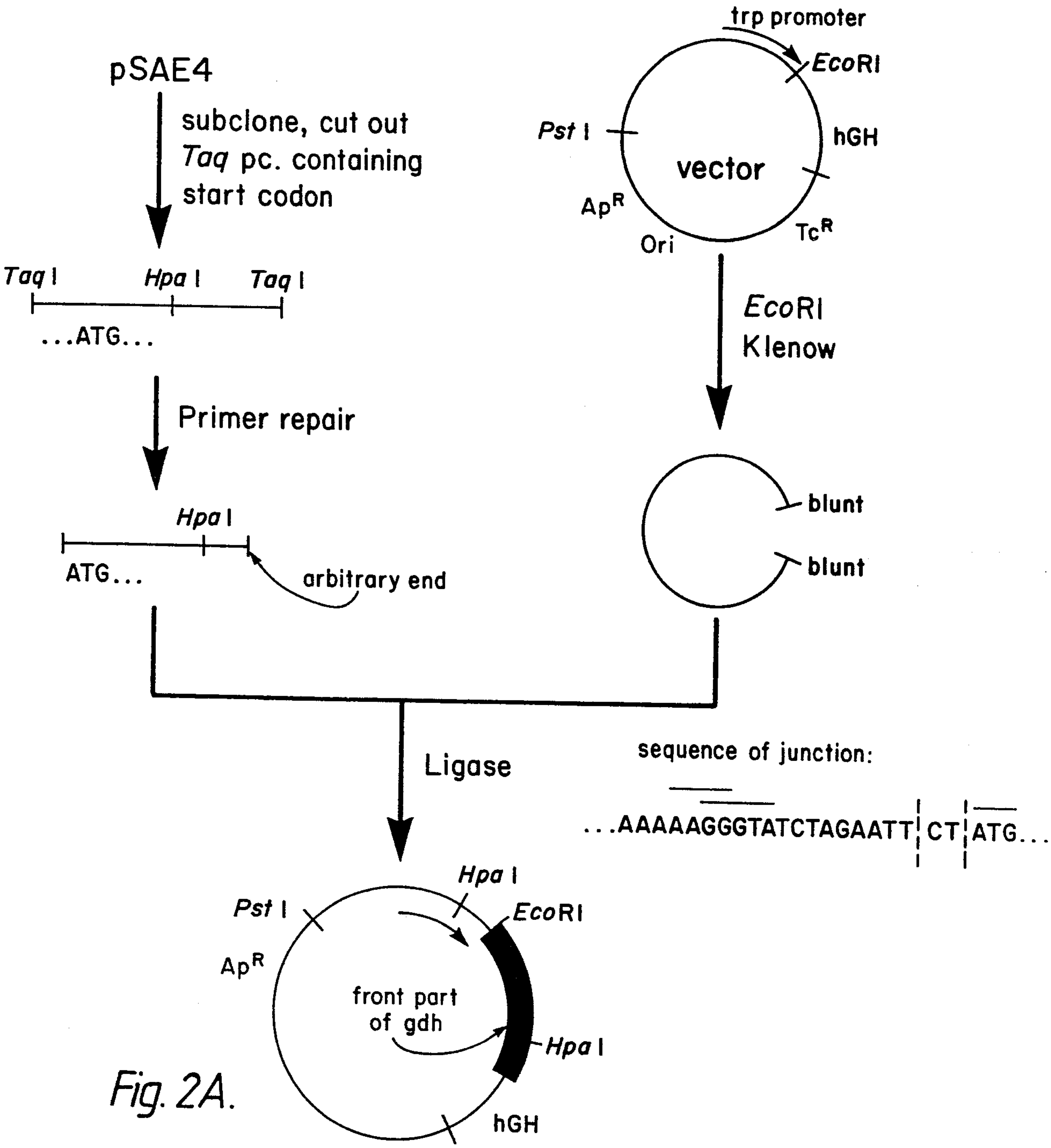
pSAE4
subclone, cut out
Taq pc. containing
start codon
Taq I
Hpa I
Taq I
...ATG...
Primer repair
Hpa I
ATG...
arbitrary end
trp promoter
EcoRI
Pst I
hGH
vector
ApR
TcR
Ori
EcoRI
Klenow
blunt
blunt
Ligase
sequence of junction:
...AAAAAGGGTATCTAGAATT CT ATG...
Hpa I
Pst I
EcoRI
ApR
front part
of gdh
Hpa I
hGH

Fig. 2A.

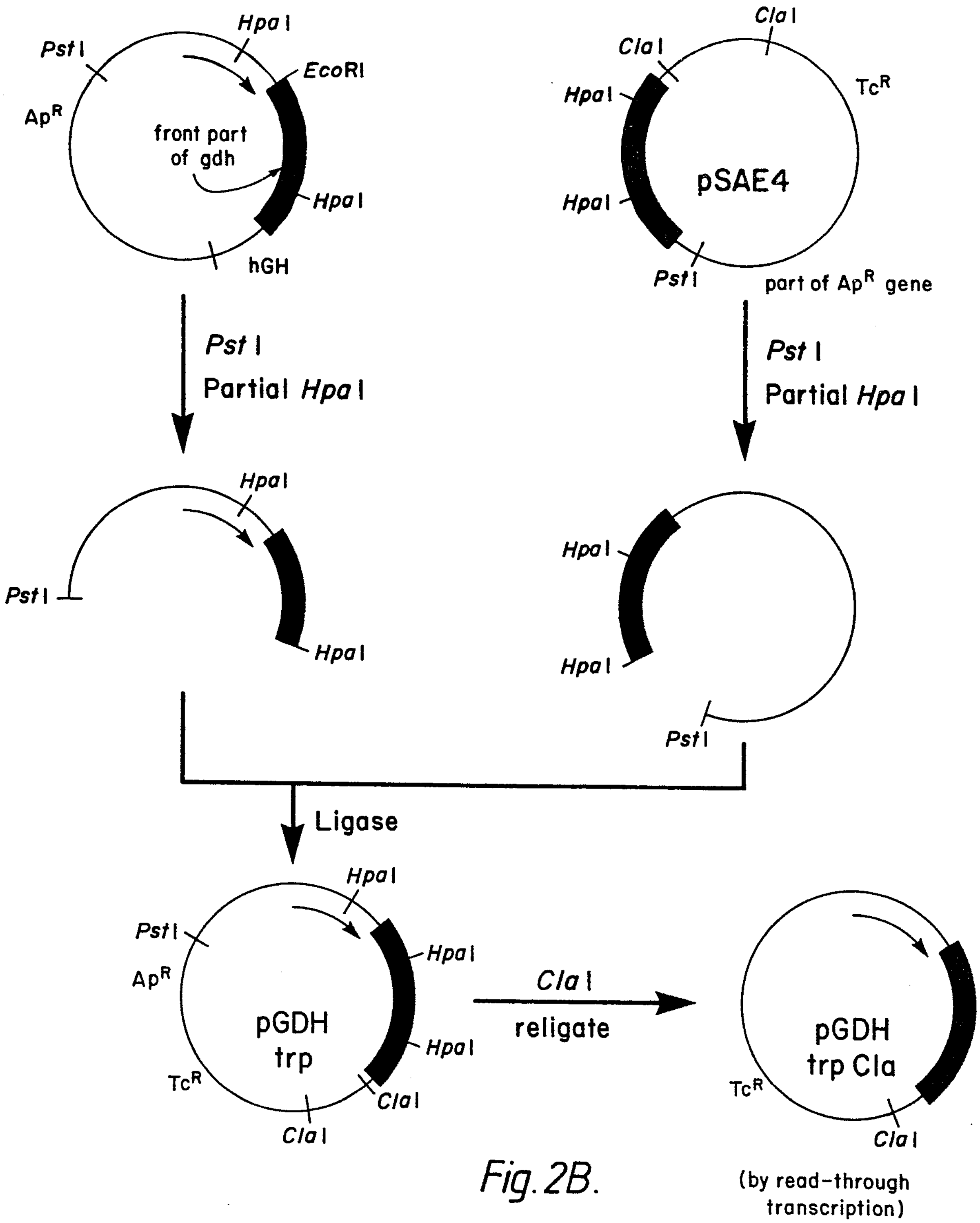
Hpa I
Pst I
EcoRI
ApR
front part
of gdh
Hpa I
hGH
Cla I
Cla I
TcR
Hpa I
pSAE4
Hpa I
Pst I
part of ApR gene
Pst I
Partial Hpa I
Pst I
Partial Hpa I
Hpa I
Pst I
Hpa I
Hpa I
Hpa I
Pst I
Ligase
Hpa I
Pst I
Hpa I
ApR
pGDH
trp
Hpa I
TcR
Cla I
Cla I
Cla I
religate
pGDH
trp Cla
TcR
Cla I
(by read-through
transcription)

Fig. 2B.

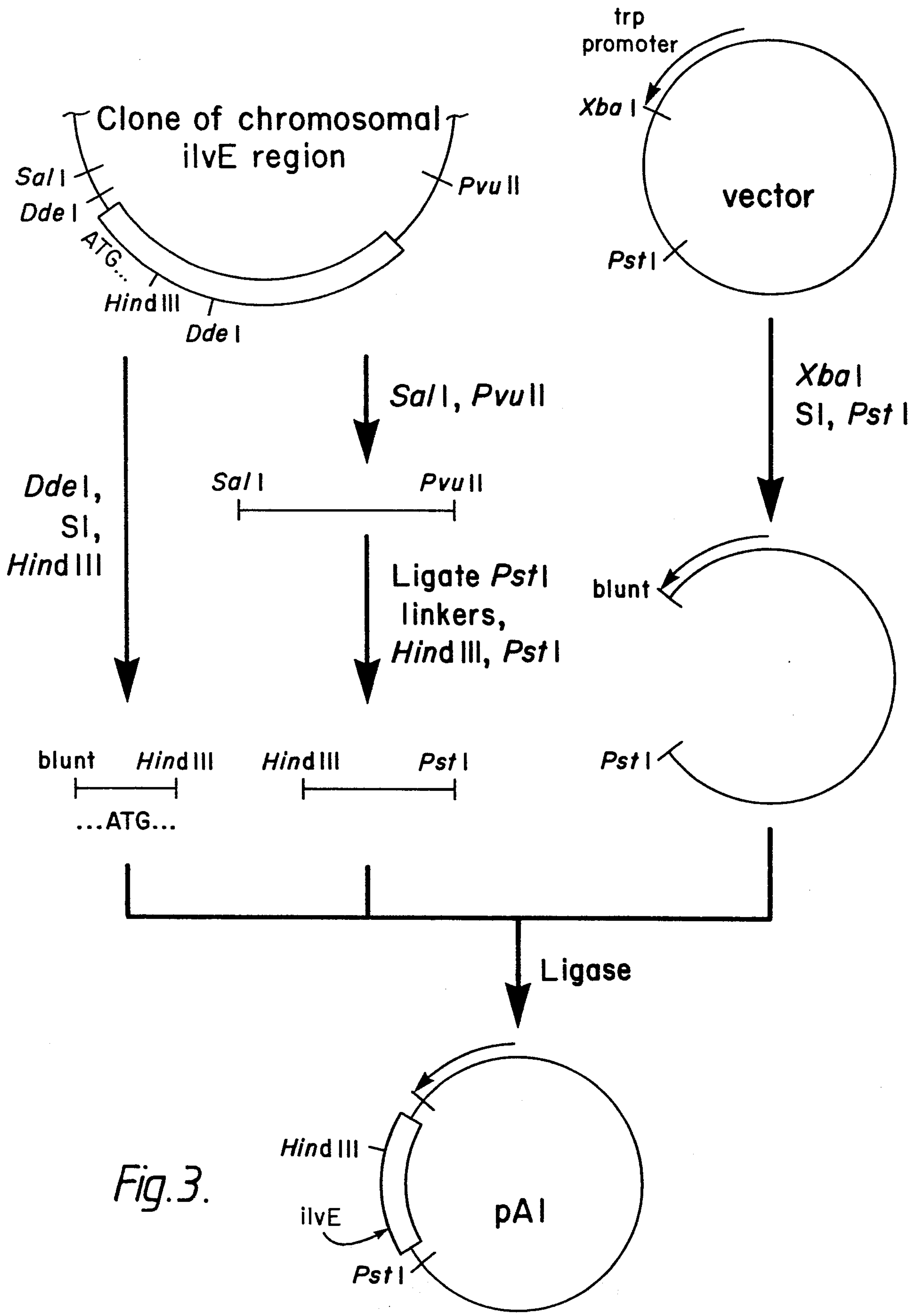
Clone of chromosomal ilvE region
Sal I
Dde I
ATG...
Hind III
Dde I
Pvu II
trp promoter
Xba I
vector
Pst I
Dde I, SI, Hind III
Sal I, Pvu II
Sal I
Pvu II
Ligate Pst I linkers, Hind III, Pst I
Xba I SI, Pst I
blunt
Pst I
blunt
Hind III
...ATG...
Hind III
Pst I
Ligase
Hind III
ilvE
Pst I
pA I

Fig.3.

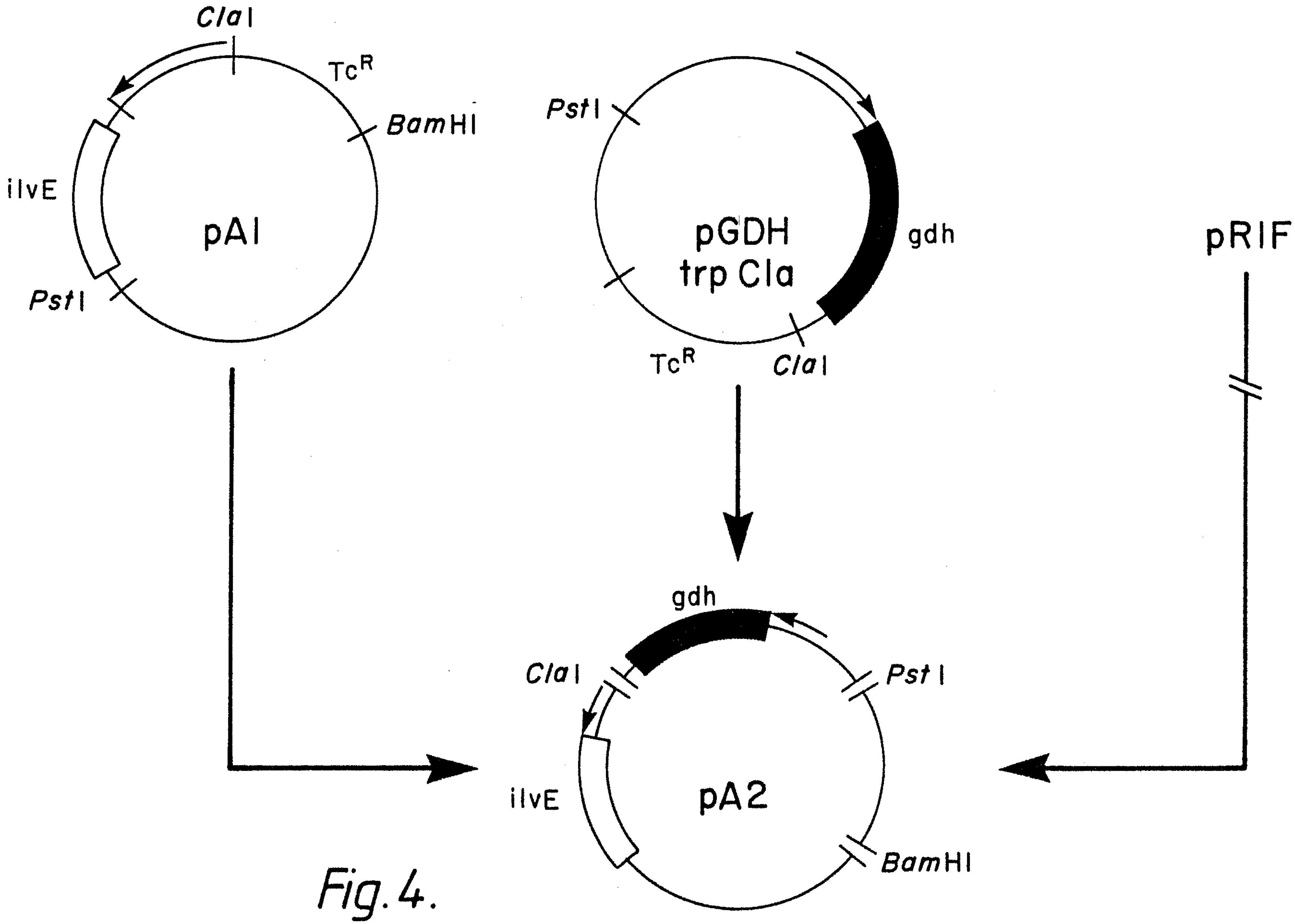

ClaI
TcR
BamHI
ilvE
pAI
PstI
PstI
pGDH
trp Cla
gdh
TcR
ClaI
pRIF
gdh
ClaI
PstI
ilvE
pA2
BamHI


Fig. 4.

0137646

European Patent Office

# EUROPEAN SEARCH REPORT

Application number

EP 84305532.8

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.4) |
|---|---|---|---|
| A | EP - A2 - 0 023 346 (DEGUSSA AKTIENGESELLSCHAFT et al.)<br>* Abstract *<br>-- | 1 | C 12 N 15/00<br>C 12 P 13/04<br>C 12 P 13/22<br>//C 12 R 1/19 |
| A | EP - A2 - 0 046 186 (BASF AKTIENGESELLSCHAFT)<br>* Claim 1 *<br>-- | 1 | |
| D,A | US - A - 4 016 037 (MITSUGI et al.)<br>* Claim 1 *<br>-- | 1 | |
| A | GB - A - 2 053 906 (AJINOMOTO CO., LTD.)<br>* Abstract *<br>---- | 1-3 | |
| | | | TECHNICAL FIELDS SEARCHED (Int. Cl.4) |
| | | | C 12 N<br>C 12 P |

The present search report has been drawn up for all claims

| Place of search | Date of completion of the search | Examiner |
|---|---|---|
| VIENNA | 23-11-1984 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document